# EUROPEAN PATENT APPLICATION

(11) **EP 4 740 790 A1**
(43) Date of publication of application: **13.05.2026**
(21) Application number: 24212281.0
(22) Date of filing: 12.11.2024
(51) Int. Cl.: A46B 15/00, A61B 5/11, A61B 5/00

(54) **BITING EVENT DETECTION AND PROTECTION FOR ORAL CLEANING DEVICES**

(71) Applicant: Koninklijke Philips N.V., 5656 AG Eindhoven (NL)
(72) Inventor: STEFAN, André Christian, Eindhoven (NL); VAN NOORD, Kris, Eindhoven (NL); SOLERIO, Daniele, Eindhoven (NL)
(74) Representative: Philips Intellectual Property & Standards

(57) **Abstract**

Disclosed herein is an oral cleaning device (100, 400) comprising a drive train (111) configured for actuating a cleaning element (118). The oral cleaning device further comprises a motion sensor (112, 112') configured for providing sensor data (132) descriptive of motion of the drive train. The oral cleaning device further comprises; a memory (128) storing machine executable instructions (130).

The oral cleaning device further comprises a computational system (126). Execution of the machine executable instructions causes the computational system to: repeatedly (200) receive the sensor data from the motion sensor; detect (202) a biting event (136) by inputting the repeatedly acquired sensor data into a biting event detection algorithm (134); and execute (204) a biting event protocol if the biting event is detected, wherein the biting event protocol comprises reducing power (138) supplied to drive train for a predetermined duration.

## Description

### TECHNICAL FIELD

The invention relates to oral cleaning devices, in particular to the protection of the drive train of an oral cleaning device during biting events.

### BACKGROUND OF THE INVENTION

Oral cleaning devices, such as electric toothbrushes or sonic toothbrushes, are designed to improve dental hygiene by utilizing motor-driven brushing techniques that enhance plaque removal and gum cleaning. These devices often incorporate features like oscillating or sonic movements and timers to ensure thorough and consistent cleaning.

United States patent publication US 10,813,733 B2 discloses a drivetrain assembly for a personal care device, the drivetrain assembly including a primary resonator; a secondary resonator configured to reduce vibrations transmitted from the motor to a body of the personal care device; a fixed point positioned between the primary resonator and the secondary resonator; a first spring member connected at a first end to the primary resonator and at a second end to the fixed point; a second spring member connected at a first end to the secondary resonator and at a second end to the fixed point; a coupling spring connected at a first end to the primary resonator and at a second end to the secondary resonator; and an actuator configured to exert force on at least one of the primary resonator and the secondary resonator.

### SUMMARY OF THE INVENTION

The invention provides for an oral cleaning device, an oral cleaning system, a method of operating an oral cleaning device, and a computer program in the independent claims. Embodiments are given in the dependent claims.

In one aspect an oral cleaning device is disclosed. The oral cleaning device comprises a drive train configured for actuating a cleaning element. The oral cleaning device further comprises a motion sensor configured for providing sensor data descriptive of the motion of the drive train. The oral cleaning device further comprises a memory storing machine-executable instructions.

The oral cleaning device further comprises a computational system. Execution of the machine-executable instructions causes the computational system to repeatedly receive the sensor data from the motion sensor. Execution of the machine-executable instructions further causes the computational system to detect a biting event by inputting the repeatedly acquired sensor data into a biting event detector algorithm. Execution of the machine-executable instructions further causes the computational system to execute a biting event protocol if the biting event is detected. The biting event protocol comprises reducing power supplied to the drive train for a predetermined duration.

In another aspect an oral cleaning system is disclosed. The oral cleaning system comprises an oral cleaning device. The oral cleaning device further comprises a radio-frequency transmitter. Execution of the machine-executable instructions further causes the computational system to transmit the biting event warning signal as a radio-frequency signal using a radio-frequency transmitter. The oral cleaning system further comprises a handheld computing device. The handheld computing device is configured to receive a biting event warning signal via the radio-frequency signal. The handheld computing device is further configured to display a predetermined educational message in response to receiving the biting event warning signal.

In another aspect, a method of operating the oral cleaning device is disclosed. The oral cleaning device comprises a drive train configured for actuating a cleaning element. The oral cleaning device further comprises a motion sensor configured for providing sensor data descriptive of motion of the drive train. The method comprises repeatedly receiving the sensor data from the motion sensor. The method further comprises detecting a biting event by inputting the repeatedly acquired sensor data into a biting event detector algorithm. The method further comprises executing a biting event protocol if the biting event is detected. The biting event protocol comprises reducing power supplied to the drive train for a predetermined duration.

In another aspect, a computer program comprising machine-executable instructions for execution by a computational system is disclosed. The computational system is configured for controlling an oral cleaning device. The oral cleaning device comprises a drive train configured for actuating a cleaning element. The oral cleaning device further comprises a motion sensor configured for providing sensor data descriptive of motion of the drive train. Execution of the machine-executable instructions causes the computational system to repeatedly receive the sensor data from the motion sensor. Execution of the machine-executable instructions further causes the computational system to detect a biting event by inputting the repeatedly acquired sensor data into a biting event detection algorithm. Execution of the machine-executable instructions further causes the computational system to execute a biting event protocol if the biting event is detected. The biting event protocol comprises reducing power supplied to the drive train for a predetermined duration.

### BRIEF DESCRIPTION OF THE DRAWINGS

In the following preferred embodiments of the invention will be described, by way of example only, and with reference to the drawings in which:
Fig. 1 illustrates an example of an oral cleaning device.
Fig. 2 shows a flow chart which illustrates a method of operating the oral cleaning device of Fig. 1.
Fig. 3 illustrates an example of an oral cleaning system.
Fig. 4 illustrates a further example of an oral cleaning device.
Fig. 5 shows a plot of an amplitude signal indicating a biting event.
Fig. 6 shows amplitude plots for the front and rear masses during a biting event.

### DESCRIPTION OF EMBODIMENTS

Like numbered elements in these figures are either equivalent elements or perform the same function. Elements which have been discussed previously will not necessarily be discussed in later figures if the function is equivalent.

In one aspect, there is an oral cleaning device that comprises a drive train configured for actuating a cleaning element. As used herein, a drive train encompasses a motor or actuator used for providing mechanical motion and linkages which connect this motor or resonator to the cleaning element. A cleaning element may be an attachment which is attached to the oral cleaning device and enables performance of a task of oral cleaning such as brushing teeth.

The oral cleaning device further comprises a motion sensor that is configured for providing sensor data descriptive of motion of the drive train. The oral cleaning device further comprises a memory storing machine-executable instructions. The oral cleaning device also comprises a computational system. Execution of the machine-executable instructions causes the computational system to repeatedly receive the sensor data from the motion sensor. For example, the sensor data may be received as it is acquired or sent in packets periodically to the computational system by the sensor system. Execution of the machine-executable instructions further causes the computational system to detect a biting event by inputting the repeatedly acquired sensor data into a biting event detection algorithm. A biting event, as used herein, encompasses when an operator of the oral cleaning device bites on the cleaning element which may cause a portion of the drive train, which is connected to the cleaning element, to have its motion blocked or greatly reduced.

The biting event detection algorithm may be implemented in various ways, as will be described below. It may for example be able to detect a pattern, look at frequency changes in the acquired sensor data or using other methods to detect when the biting event occurs. Execution of the machine-executable instructions further causes the computational system to execute a biting event protocol if the biting event is detected. The biting event protocol comprises reducing power supplied to the drive train for a predetermined duration. When a biting event occurs, this may cause a portion of the drive train to have its motion reduced or greatly damped or limited. This for example may then result in mechanical damage to one or more components or parts of the drive train. By reducing the power for a predetermined duration, this may enable the drive train to reduce or avoid mechanical damage. For example, when the operator of an oral cleaning device notices that the amplitude or power produced by the oral cleaning device is suddenly reduced, this may cause the operator to stop biting the cleaning element. During the predetermined duration various tasks may also be additionally performed by the computational system. For example, a warning signal or indication may be presented to the operator to warn that the oral cleaning device is being bitten or provide instructions so the operator stops biting the cleaning element.

In another example, the biting protocol is configured to prevent or reduce mechanical damage to the drive train during the biting event. When an operator or user of the oral cleaning device bites down on the cleaning element this may, as was mentioned before, cause portions of the drive train to have their amplitude reduced or have their motion stopped. This may then cause additional mechanical damage in other parts of the drive train. This example is advantageous because it may for example be used to reduce the amount of mechanical damage to the drive train during a biting event.

In another example the drive train comprises a mechanical resonator. The mechanical resonator comprises a front mass configured for oscillating about an axis or rotation. The front mass is configured for actuating the cleaning element. The sensor data is descriptive of motion of the front mass. The mechanical resonator further comprises a rear mass configured for oscillating about the axis of rotation. The mechanical resonator further comprises an oscillatory linkage spring configured for coupling oscillatory motion between the rear mass and the front mass. The mechanical resonator further comprises an electromagnetic oscillating system configured for driving oscillation of the rear mass. The reduction of power supplied to the drive train for a predetermined duration comprises reducing power to the electromagnetic oscillating system for the predetermined duration.

In this example, the drive train uses a mechanical resonator to provide the oscillatory motion of the cleaning device. An advantage of this mechanical resonator is that it is able to actuate the cleaning element with a reduced amount of power. A potential difficulty is that if there is a biting event this may cause the front mass to have its motion reduced or stopped even altogether in some examples. The energy from the electromagnetic oscillating system poured into the rear mass may then potentially build up and cause mechanical damage to the mechanical resonator. Reducing the power to the electromagnetic oscillating system for the predetermined duration during the biting event may prevent damage to the rear mass and/or other mechanical components of the mechanical resonator.

In another example, the drive train comprises a driveshaft configured for receiving the cleaning element. The sensor data is descriptive of a time-dependent rotational position of the cleaning element or of the front mass. The biting event detection algorithm is configured to determine an amplitude signal by repeatedly fitting the sinusoidal function to the sensor data within a sliding temporal window. The biting event detection algorithm is configured to detect a decrease in amplitude of the motion of the driveshaft using the amplitude signal. In this particular example, the motion sensor is able to detect the oscillatory motion of the front mass or the cleaning element. An amplitude of this oscillatory motion is calculated using the sliding window or on the fly by repeatedly fitting a sinusoidal signal to the sensor data. This enables very accurate and rapid determination of the amplitude. This may have the benefit then of enabling the biting event detection algorithm to accurately predict when a biting event is occurring.

In another example, the biting event detection algorithm is configured to detect the biting event by detecting a decrease in the amplitude signal above a predetermined threshold. In other words, when the amplitude signal decreases above a certain amount, this may trigger the biting event. This may provide for a very simple and accurate means of detecting when a biting event is occurring.

In another example the biting event detection algorithm is configured to detect changes in the amplitude signal within a moving temporal window using any one of the following: calculating a standard deviation of the amplitude signal within the moving temporal window and detecting the biting event by detecting a predetermined increase in the amplitude signal with respect to the calculated standard deviation, calculating a mean of the amplitude signal within the moving temporal window and detecting the biting event by detecting a predetermined decrease in the amplitude signal with respect to the calculated mean, and applying a digital filter to the amplitude signal within the moving temporal window and detecting the biting event if the output of the digital signal is outside predetermined thresholds. These various examples may provide an accurate determination of when the biting event may be occurring. It is also noted that applying a digital filter also encompasses applying more than one digital filter. As is described below, an additional embodiment is to calculate a Fourier transform and then to check if certain frequency bins of the frequency spectrum have had a predetermined change. The same effect of looking at several different bin frequency spectrums may be achieved by applying multiple digital filters to look at how the component of the amplitude signal within certain frequency ranges or bins is occurring. In other words, instead of applying a Fourier transform and then observing the change in several frequency bins, several different digital filters can be applied and the output of the several different digital filters may be monitored.

In another example, the biting event detection algorithm comprises a machine learning algorithm configured to detect the biting event in the sensor data.

A variety of machine learning algorithms could be used to implement the biting event detection algorithm. One example would be various classical machine learning algorithms such as Support Vector Machine, k-Nearest Neighbor, Naive Bayes, and Decision Trees. The training process of classical ML models generally involves two stages: first, data preprocessing, where feature engineering and cleaning play a critical role. Second, the model is trained using algorithms that minimize a predetermined loss function. These models are trained based on well-defined features. The biting event is triggered when the ML model, trained on specific data, detects it.

Another class of machine learning models that could be used to implement the biting event detection algorithm would be Ensemble ML algorithms such as Random Forest Classifier or Gradient Boosting Classifier. The training process of Ensemble ML models consists of independently trained base models. The ensemble model is created by combining the outputs of each base model that has been trained using a particular algorithm or technique. The base model predictions are aggregated using methods such as bagging, boosting, or stacking. In order to make up for the shortcomings of each individual model, this process makes use of the diversity of the base models to produce predictions that are more accurate overall. The biting event is triggered when the Ensemble ML model, trained on specific data, detects it.

A further option for implementing the biting event detection algorithm using machine learning would be the use of Artificial Neural Networks (ANN) such as Feedforward Neural Network, Convolutional Neural Network, and Recurrent Neural Networks. The training process of Artificial Neural Networks (ANNs) involves a different methodology, as it uses both forward and backward propagation. First, information is sent over the neural network, and the result is compared with the actual target. The model's weights and biases are then modified by backpropagating errors using optimization techniques like stochastic gradient descent. Iterative training is used until the model converges to a performance level that is acceptable. The biting event is triggered when the ANN model, trained on specific data, detects it.

Recurrent neural networks (RNN) may be particularly suited as they are able to learn patterns as well as to perform signal processing tasks. RNNs, particularly their variations like Long Short-Term Memory (LSTM) or Gated Recurrent Unit (GRU) may work well as they can capture temporal dependencies and remember prior amplitude values over time.

Some example RNN Architectures which could be used include Simple RNNs. This can be the starting point, where a series of amplitude data is fed into the network, and each hidden state depends on the previous one. An alternative RNN architecture that may be used is the above mentioned LSTM. An LSTM-based architecture can handle long sequences of data more effectively by controlling the flow of information with gates (input, forget, and output). This would be useful if the decrease in amplitude occurs over an extended time or if small, subtle changes are important. A further RNN architecture which may be used is the GRU architecture. A GRU is a simplified version of LSTM, requiring less computational power but still capturing long-term dependencies. It can work well for the amplitude data if the pattern of biting is straightforward. The reduced computational power may make a GRU architecture attractive for implementation on an embedded system or computational system of an oral cleaning device.

An RNN could be trained using a labeled dataset where the input is the amplitude sequence and the output is a binary label indicating whether a biting event has occurred or not at various time steps. In some examples, a sliding window approach could be used where each window captures a few time steps of amplitude data and feed it to the network. For loss function, a binary cross-entropy loss function could be appropriate.

Machine learning may be used to detect the changes in the amplitude using several different ways. For example, the samples for a predetermined period of time may be input into the neural network and a pattern may be detected. In other examples, the machine learning algorithm may for example be used in place of a digital filter or digital transform. In this example, a recurrent neural network may be used for performing digital signal processing and detecting when various frequency components change.

In another example, the biting event detection algorithm is configured to repeatedly calculate a Fourier transform of the sensor data. The calculated Fourier transform comprises a bin frequency spectrum. The biting event detection algorithm is further configured to detect a biting event by detecting changes in at least one predetermined bin of the bin frequency spectrum. This may be beneficial because when the cleaning element it bit down upon by an operator or user, it may cause a change in the mechanical structure or resonances which are present in the drive train. Biting on the cleaning element may therefore be detectable by looking at the binned frequency spectrum.

In another example, the drive train comprises a driveshaft configured for receiving the cleaning element. The oral cleaning device comprises a reflective element attached to the driveshaft. The oral cleaning device comprises a body portion for housing the drive train. The sensor is an optical sensor. The optical sensor comprises the one light source and multiple light sensors. The optical sensor has a fixed position relative to the body portion. The at least one light source is optically coupled to the multiple light sensors via the reflective element. This example may be beneficial because it provides for an effective and accurate means of measuring sensor data during a biting event.

In another example, the biting event protocol comprises providing a biting event warning signal. This warning signal may be provided in several different ways. For example, the oral cleaning device itself may have a warning indicator which is able to provide a biting event warning signal. In other examples, radio-frequency or other signals may be used to transmit a warning signal to another device, such as a handheld computing device.

In another example, the oral cleaning device comprises a warning indicator. The warning indicator may for example provide a text, an audio, or visual indication to the operator or user of the oral cleaning device. Execution of the machine-executable instructions causes the computational system to indicate the biting event warning signal using the warning indicator. For example, when a biting event is detected, an optical warning indicator may flash a visible warning. In other examples there may be an audible warning indicator such as a tone or beeping sound which is used to indicate the biting event warning.

In another example the oral cleaning device comprises a radio-frequency transmitter. Execution of the machine-executable instructions further causes the computational system to transmit the biting event warning signal as a radio-frequency signal using the radio-frequency transmitter. This may be beneficial in several respects. In one aspect this warning signal could be used to trigger another device to provide a more complicated warning or instructions to the operator or user of the oral cleaning device. In other examples, the biting event warning signal could for example be logged by a different device and used when diagnosing failures or defects in the oral cleaning device.

In another aspect, there is an oral cleaning system. The oral cleaning system comprises an oral cleaning device and a handheld computing device. The handheld computing device may for example be a battery powered smartphone or other handheld computing device. The handheld computing device is configured to receive the biting event warning signal via the radio-frequency signal and then display a predetermined educational message in response to receiving the biting event warning signal. This for example may be useful because it may provide a means of providing more detailed instructions to the operator to stop biting the cleaning element and may also provide for extended use of the oral cleaning system as the operator is using the device more effectively.

In another aspect, there is a method of operating an oral cleaning device. The oral cleaning device comprises a drive train configured for actuating a cleaning element. The oral cleaning device further comprises a motion sensor configured for providing sensor data descriptive of the motion of the drive train.

The method comprises repeatedly receiving the sensor data from the motion sensor. The method further comprises detecting a biting event by inputting the repeatedly acquired sensor data into a biting event detection algorithm. The method further comprises executing a biting event protocol if the biting event is detected. The biting event protocol comprises reducing power supplied to the drive train for a predetermined duration. The benefits of this method have been previously discussed.

In another example, there is a computer program comprising machine-executable instructions for execution by a computational system that is configured for controlling an oral cleaning device. The oral cleaning device comprises a drive train configured for actuating a cleaning element. The oral cleaning device further comprises a motion sensor configured for providing sensor data descriptive of the motion of the drive train.

Execution of the machine-executable instructions causes the computational system to repeatedly receive the sensor data from the motion sensor. Execution of the machine-executable instructions further causes the computational system to detect a biting event by inputting the repeatedly acquired sensor data into a biting event detection algorithm. Execution of the machine-executable instructions further causes the computational system to execute a biting event protocol if the biting event is detected. The biting event protocol comprises reducing power supplied to the drive train for a predetermined duration.

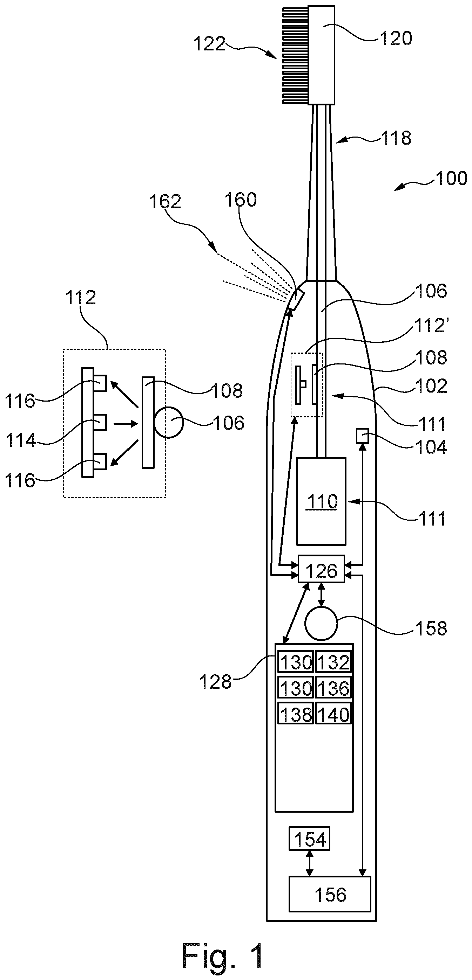

Fig. 1 illustrates an example of an oral cleaning device 100. In this example the oral cleaning device 100 is an electric toothbrush. The oral cleaning device comprises a body portion 102 which forms a handle which an operator can use to hold the oral cleaning device 100. Element 104 is an optional radio-frequency transmitter that may be used to send or receive signals to other devices such as a handheld computing device.

The oral cleaning device 100 further comprises a drive shaft 106. The drive shaft 106 is connected to a mechanical drive 110 which is used to actuate or move the drive shaft 106. The driveshaft 106 and the mechanical driver 110 form a drive train 111. The mechanical drive 110 may in some examples be a motorized system or in others it may be a mechanical resonator.

The oral cleaning device is shown as comprising a motion sensor 112 both as a top view 112 and as a side view 112'. In this example, the motion sensor 112 is an optical sensor.

The motion sensor in this example comprises a reflective element 108 optionally mounted on the drive shaft 106. The optical sensor 112 is made up of at least one light source 114 and multiple light sensors 116. The reflective element 108 is used to couple light from the light source 114 into the light sensors 116. Changes in the measured amount of light measured by the light sensors 116 are used to measure a deflection or torsion of the mechanical drive 110 and thereby measure a mechanical load on the mechanical drive 110.

The drive shaft 106 is shown as coupling to a cleaning element 118 which in this case is a toothbrush. The cleaning element 118 comprises a brush head 120 with a bristle face 122. Force or torque on the bristle face 122 by an operator will also transmit this torque or force to the drive shaft 106. The optical sensor 112 can optionally measure this torsion or displacement and determine a torque or force from it. The optical sensor 112, 112' may for example be arranged such that when a forces is exerted on the bristle face 122 it causes the distance between the light sensors 116 and the reflective element 108 to increase.

The oral cleaning device 100 is shown as comprising a computational system 126 that is configured for operating and controlling the oral cleaning device 100. It is in communication with the various electronic components of the oral cleaning device 100. The computational system 126 is in communication with a memory 128. The memory 128 is intended to represent various types of memory or data storage accessible to the computational system 126. The memory 128 is shown as containing machine-executable instructions 130. The machine-executable instructions 130 enable the computational system 126 to perform basic numerical and data processing tasks as well as control of the oral cleaning device 100.

The memory 128 is shown as storing machine-executable instructions 130. The machine-executable instructions 130 enable the computational system 126 to perform tasks such as controlling the operation and function of the oral cleaning device 100 as well as performing various mathematical and numerical tasks used in the process of controlling the oral cleaning device 100.

The memory 128 is further shown as storing sensor data 132 that has been acquired from the sensor 112. The memory The memory 128 is further shown as storing a biting event detection algorithm 134. The memory 128 is further shown as storing an indication of a biting event 136 that was output by biting event detection algorithm 134 in response to receiving sensor data 132 that contained or was descriptive of a biting event. The memory 128 is further shown as containing power reduction commands 138 which are used to reduce power to the mechanical drive 110 for a predetermined duration. These commands 138 can be part of a biting event protocol. The memory 128 is further shown as containing an optional warning signal 140 that may be used to trigger further operations of the computational system 126 or may even be transmitted via the radio-frequency transmitter 104. The oral cleaning device 100 is further shown as comprising an optional battery 154 and optional charging system 156. The optional charging system 156 can for example be used with an external charging unit 170. The external charging unit 170 could function as a base which supports the oral cleaning device in a vertical direction. The charging system 156 could also be used to detect if the oral cleaning device 100 is not being used and is therefore in a stationary state. For example, when the battery 154 is being charged by the charging system 156 then it would be clear that the oral cleaning device 100 would be stationary and mounted to the external charging unit 170.

The oral cleaning device 100 may also have an optional control 158 that enables an operator to turn on/off or operate the oral cleaning device 100 as well as an optional indicator 160 that provides an optical (light) signal 162. The optical signal 162 may be an optical warning signal used to indicate when a biting event is detected.. The indicator 160 may for example display different colors of light 162 indicating the amount of force or torque being exerted on the cleaning element 118 or when a biting event occurs. For example, green may indicate that both the torque and force are at acceptable levels, yellow may be used to indicate a boundary region where the operator should try to decrease the amount of force or torque, and red may indicate a level of force or torque which may injure or not be conducive for cleaning. A biting event could for example be indicated by a flashing optical signal.

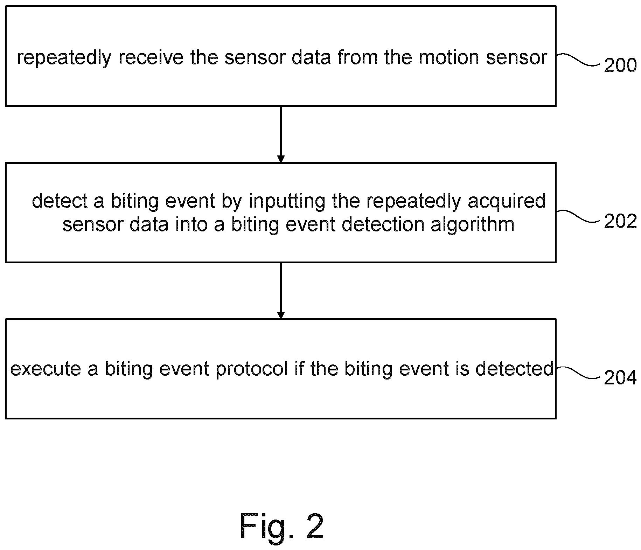

Fig. 2 shows a flowchart which illustrates a method of operating the oral cleaning device 100. In step 200, the sensor data 132 is repeatedly received from the motion sensor 112, 112'. In step 202, the biting event 136 is detected by inputting the repeatedly acquired sensor data 132 into the biting event detection algorithm 134. In step 204, the biting event protocol is executed. This is performed if the indication of the biting event 136 is provided. The execution of this protocol comprises executing the power reduction commands 138 which cause a reduction of power to the mechanical drive 110 for the predetermined duration.

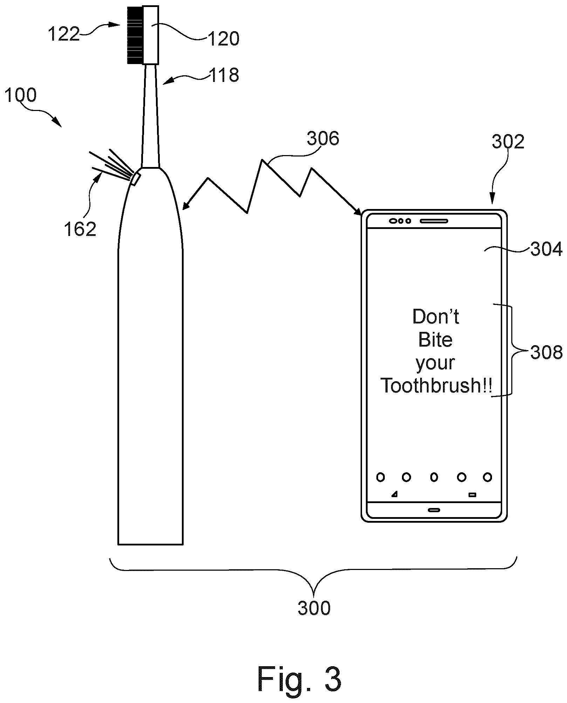

Fig. 3 illustrates an example of an oral cleaning system 300. The oral cleaning system 300 comprises the oral cleaning device 100 and a handheld computing device 302. The handheld computing device 302 comprises a display 304. In this example the oral cleaning device 100 has detected a biting event and transmits a radio-frequency transmission 306 that contains the biting event warning signal 140. The reception of the biting event warning signal 140 causes the handheld computing device to display a predetermined educational message 308 on the display 304. This for example could be used to convey detailed information about easing the biting of the cleaning element 118 as well as providing further motivation such as explaining why the cleaning element 118 should not be bit.

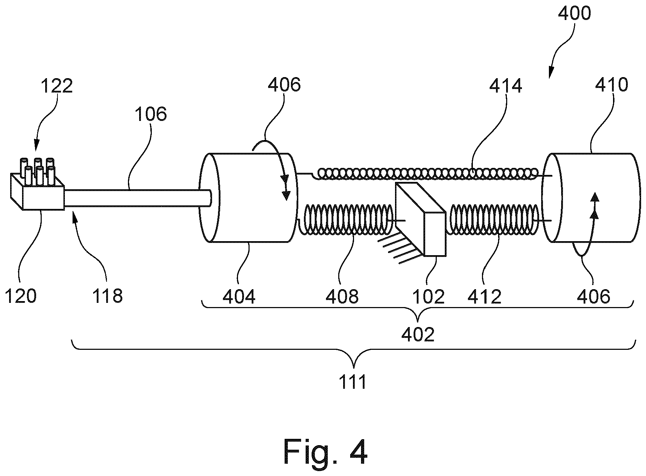

Fig. 4 illustrates an idealized diagram of an oral cleaning device 400. This simplified diagram illustrates the functioning of a mechanical resonator 402, which is used as the mechanical drive 110. The brush head is connected to the driveshaft 106. The driveshaft 106 and the mechanical resonator 402 form the drive train 111. The driveshaft 106 is connected to the front mass 404. The front mass 404 is configured for rotational motion about a rotational axis 406. It is further coupled using a front mass spring 408 to the frame or body portion 102 of the oral cleaning device 400. The front mass 404 is used to drive the shaft 106 and thereby the cleaning element 118.

There is also a rear mass 410 which also rotates about the rotational axis 406. It is coupled via a rear mass spring 412 to the body portion 102 also. There is an oscillatory linkage spring 414 which is used to couple rotational motion between the front mass 406 and the rear mass 410. Though it is not shown in this diagram, there is an electromagnetic oscillating system which drives the rotational motion of the rear mass 410. The oscillatory linkage spring 414 is then used to couple rotational motion to the front mass 404. The frequency which the electromagnetic oscillating system uses to drive the rear mass 410 is chosen such that the oscillatory motion has the front mass 406 and the rear mass 410 moving in opposite rotational directions. This cancels the torque of the two masses 404, 410.

An advantage of this mechanical resonator 402 is that a minimal amount of energy is needed to drive the cleaning element 118. If the operator or user of the oral cleaning device 400 bites down on the cleaning element 118 this will change the mechanical coupling of the system. This may cause the front mass 404 to stop rotating or be severely damped. A possible result of this is that the rear mass 410 may have its rotational amplitude greatly increased. This may for example result in mechanical damage to various components such as the rear mass spring 412.

In the arrangement illustrated in Fig. 1, the motion sensor 112 is able to detect the rotational amplitude of the driveshaft 106 and also equivalently, the rotational amplitude of the front mass 404. When a sufficient decrease in the amplitude is detected or a biting event is detected, the power to the electrode oscillating system is greatly reduced and this reduces the amount of energy used to drive the rear mass 410, thereby preventing mechanical damage.

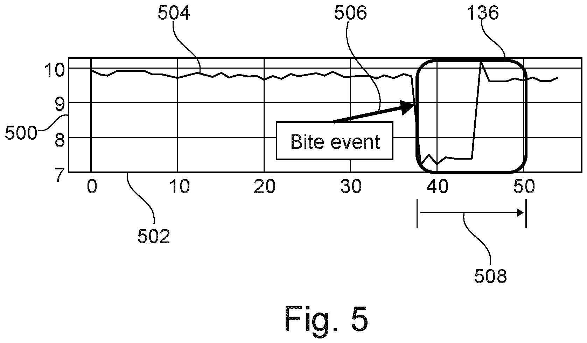

Fig. 5 shows the plot of the amplitude signal 504 from the motion sensor 112. The amplitude signal 504 is applied as a function of amplitude 500 in degrees versus time 502. At event 506 there is a rapid decrease in the amplitude 500. This is then identified as a biting event 136. The interval 508 may for example be an exemplary predetermined duration 508 where the power to the mechanical drive 110 may be decreased. If the power to the mechanical drive 110 lasts the predetermined duration 508, as is illustrated in this example, it may be able to prevent mechanical damage. In the particular case illustrated in Fig. 5, experiments show that the amplitude signal 504 decreased from 9.9 degrees peak to peak to below 8 degrees peak to peak during a biting event 136. One algorithm that could be used to detect a biting event, and would work in this case, would be to trigger the biting event protocol if the amplitude is reduced by two degrees peak to peak (or some other predetermined threshold).

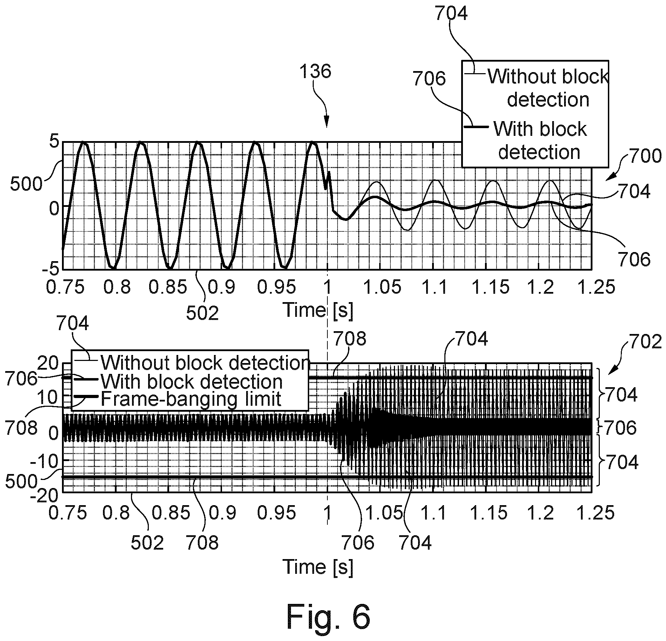

Fig. 6 illustrates how examples may be used to prevent damage to the mechanical components coupled to the rear mass 410. Fig. 6 shows two charts. The top chart is the amplitude of the front mass rotation 600 and 602 is the amplitude of the rear mass rotation. These are given as the amplitude 500 as a function of degrees and the x coordinate 502 is the time in seconds. At time = 1 the figures 600, 602 indicates the beginning of a biting event 136. At the start of the biting event 136, the power provided to the electromagnetic oscillating system is reduced. These charts show two different functions. Curve 604 indicates the amplitude when there is no power reduction to the electromagnetic oscillating system. Curve 606 indicates the amplitude when the power is reduced to the electromagnetic oscillating system. In the top chart 600, it can be seen that the amplitude 604 is greater than the amplitude 606 during the biting event if the power is not reduced. This has only a minimal effect on the front mass 404.

The bottom Figure 602 illustrates that there is a dramatic effect on the amplitude of the rear mass 410 depending if the power is reduced to the electromagnetic oscillating system or not. The horizontal lines 608 indicate rotational positions which result in a frame banging limit or when mechanical components begin hitting a frame which supports the rear mass 410. This Figure illustrates that the motion 604 when there is no power reduction, exceeds the mechanical limit 608. When power is reduced to the electromagnetic oscillating system 606 then the amplitude stays within acceptable levels and there is no risk of mechanical damage.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,
Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: oral cleaning device
- 102: body portion
- 104: radio-frequency transmitter
- 106: drive shaft
- 108: reflective element
- 110: mechanical drive
- 111: drive train
- 112: motion sensor (top view)
- 112': motion sensor (side view)
- 114: light source
- 116: light sensor
- 118: cleaning element (tooth brush)
- 120: brush head
- 122: bristle face
- 126: computational system
- 128: memory
- 130: machine executable instructions
- 132: sensor data
- 134: biting event detection algorithm
- 136: indication of biting event
- 138: power reduction commands for predetermined duration
- 140: warning signal
- 154: battery
- 156: charging system
- 158: control
- 160: indicator
- 162: optical warning signal
- 200: repeatedly receive the sensor data from the motion sensor
- 202: detect a biting event by inputting the repeatedly acquired sensor data into a biting event detection algorithm
- 204: execute a biting event protocol if the biting event is detected
- 300: oral cleaning system
- 302: handheld computing device
- 304: display
- 306: radio frequency signal containing biting event warning signal
- 308: predetermined educational message
- 400: oral cleaning device
- 402: mechanical resonator
- 404: front mass
- 406: rotational about rotational axis
- 408: front mass spring
- 410: rear mass
- 412: rear mass spring
- 414: oscillatory linkage spring
- 500: amplitude
- 502: time
- 504: amplitude signal
- 506: rapid decrease in amplitude
- 508: predetermined duration
- 600: amplitude of front mass rotation
- 602: amplitude of rear mass rotation
- 604: amplitude when power is not reduced during biting event
- 606: amplitude when power is reduced during biting event

## Claims

1. An oral cleaning device (100, 400) comprising:
- a drive train (111) configured for actuating a cleaning element (118);
- a motion sensor (112, 112') configured for providing sensor data (132) descriptive of motion of the drive train;
- a memory (128) storing machine executable instructions (130);
- a computational system (126), wherein execution of the machine executable instructions causes the computational system to:
- repeatedly (200) receive the sensor data from the motion sensor;
- detect (202) a biting event (136) by inputting the repeatedly acquired sensor data into a biting event detection algorithm (134); and
- execute (204) a biting event protocol if the biting event is detected, wherein the biting event protocol comprises reducing power (138) supplied to drive train for a predetermined duration.

2. The oral cleaning device of claim 1, wherein the biting protocol is configured to prevent or reduce mechanical damage to drive train during the biting event.

3. The oral cleaning device of claim 1 or 2, wherein the drive train comprises a mechanical resonator (402), wherein the mechanical resonator comprises:
- a front mass (404) configured for oscillating about an axis of rotation (406), wherein the front mass is configured for actuating the cleaning element, wherein the sensor data is descriptive of motion of the front mass;
- a rear mass (410) configured for oscillating about the axis of rotation;
- an oscillatory linkage spring (414) configured for coupling oscillatory motion between the rear mass and the front mass; and
- an electromagnetic oscillating system configured for driving oscillation of the rear mass, wherein reducing power supplied to drive train for a predetermined duration comprises reducing power to the electromagnetic oscillating system for the predetermined duration.

4. The oral cleaning device of any one of the preceding claims, wherein the sensor data is descriptive of a time dependent rotational position of the cleaning element, wherein the biting event detection algorithm is configured to determine an amplitude signal (504) by repeatedly fitting a sinusoidal function to the sensor data within a sliding temporal window, wherein the biting event detection algorithm is configured to detect a decrease in amplitude of the motion of the drive shaft using the amplitude signal.

5. The oral cleaning device of claim 4, wherein biting event detection algorithm is configured to detect the biting event by detecting a decrease in the amplitude signal (506) above a predetermined threshold.

6. The oral cleaning device of claim 4, wherein the biting event detection algorithm is configured to detect changes in the amplitude signal within a moving temporal window using and one of the following:
- calculating a standard deviation of the amplitude signal within the moving temporal window and detecting the biting event by detecting a predetermined increase in the amplitude signal with respect to the calculated standard deviation;
- calculating a mean of the amplitude signal within the moving temporal window and detecting the biting event by detecting a predetermined decrease in the amplitude signal with respect to the calculated mean; and
- applying a digital filter to the amplitude signal within the moving temporal window and detecting the biting event if the output of the digital signal is outside predetermined thresholds.

7. The oral cleaning device of any one of claims 1 through 4, wherein the biting event detection algorithm comprises a machine learning algorithm configured to detect the biting event in the sensor data.

8. The oral cleaning device of any one of claims 1 through 4, wherein the biting event detection algorithm is configured to:
- repeatedly calculate a Fourier transform of the sensor data, wherein the calculated Fourier transform comprises a binned frequency spectrum; and
- detect the biting event by detecting changes in at least one predetermined bin of the binned frequency spectrum;

9. The oral cleaning device of any one of the preceding claims, wherein the drive train comprises a drive shaft (106) configured for receiving the cleaning element, wherein the oral cleaning device comprises a reflective element (108) attached to the drive shaft; wherein the oral cleaning device comprises a body portion (102) for housing the drive train, wherein the sensor is an optical sensor, wherein the optical sensor comprises at least one light source (114) and multiple light sensors (116), wherein the optical sensor has a fixed position relative to the body portion, wherein the at least one light source is optically coupled to the multiple light sensors via the reflective element.

10. The oral cleaning device of any one of the preceding claims, wherein
the biting event protocol comprises providing a biting event warning signal (140).

11. The oral cleaning device of claim 10, wherein the oral cleaning device comprises a warning indicator (160), wherein execution of the machine executable instructions causes the computational system to indicate the biting event warning signal using the warning indicator.

12. The oral cleaning device of claim 10 or 11, wherein the oral cleaning device comprises a radio frequency transmitter (104), wherein execution of the machine executable instructions further causes the computational system to transmit the biting event warning signal as a radio frequency signal (306) using the radio frequency transmitter.

13. An oral cleaning system (300), comprising the oral cleaning device of claim 12 and a handheld computing device (302), wherein the handheld computing device is configured to:
- receive the biting event warning signal via the radio frequency signal; and
- display a predetermined educational message (308) in response to receiving the biting event warning signal.

14. A method of operating an oral cleaning device (100, 400), wherein the oral cleaning device comprises:
- a drive train (111) configured for actuating a cleaning element; and
- a motion sensor (112, 112') configured for providing sensor data descriptive of motion of the drive train;
wherein the method comprises:
- repeatedly (200) receiving the sensor data from the motion sensor;
- detecting (202) a biting event (136) by inputting the repeatedly acquired sensor data into a biting event detection algorithm (134); and
- executing (204) a biting event protocol if the biting event is detected, wherein the biting event protocol comprises reducing power (138) supplied to drive train for a predetermined duration.

15. A computer program comprising machine executable instructions (130) for execution by a computational system (126) controlling an oral cleaning device (100, 400), wherein the oral cleaning device comprises:
- a drive train (111) configured for actuating a cleaning element (118); and
- a motion sensor (112, 112') configured for providing sensor data (132) descriptive of motion of the drive train;
wherein execution of the machine executable instructions causes the computational system to:
- repeatedly (200) receive the sensor data from the motion sensor;
- detect (202) a biting event (136) by inputting the repeatedly acquired sensor data into a biting event detection algorithm; and
- execute (204) a biting event protocol if the biting event is detected, wherein the biting event protocol comprises reducing power (138) supplied to drive train for a predetermined duration.
